# EUROPEAN PATENT SPECIFICATION

(11) **EP 0 434 625 B1**
(45) Date of publication and mention of the grant of the patent: **19.04.1995**
(21) Application number: 90810987.9
(22) Date of filing: 13.12.1990
(51) Int. Cl.: A61K 31/56, A61K 38/27

(54) **IGF-I for treating adverse effect of steroid therapy**
IGF-I zur Behandlung von Nebeneffekten der Steroidtherapie
IGF-I pour le traitement des effets secondaires dans le traitement par stéroides

(30) Priority: 22.12.1989 US 455215
(43) Date of publication of application: 26.06.1991
(73) Proprietor: CIBA-GEIGY AG, 4002 Basel (CH)
(72) Inventor: Goldberg, Ronald L., Verona, NJ 07044 (US)

(56) References cited:
- EP-A- 0 308 386
- WO-A-88/03409
- WO-A-89/11293
- R. BERKOW et al.: "The Merck Manual of Diagnosis and Therapy", 15th edition,1987, pages 2500-2506, Merck & co., Inc. Rahway, NJ, US.
- BIOCHEMICAL AND BIOPHYSICAL RESEARCH COMMUNICATIONS, vol. 165, no. 1, 30thNovember 1989, pages 355-359, Academic Press, Inc.; A. PENHOAT et al.:"Synergistic effects of corticotropin and insulin-like growth factor I oncorticotropin receptors and corticotropin responsiveness in cultured bovineadrenocortical cells"
- ENDOCRINE RESEARCH, vol. 15, no. 3, 1989, pages 303-322, Marcel Dekker, Inc.;E. HEATH-MONNIG et al.: "Sensitization of human fibroblasts to insulin-likegrowth factor I by serum deprivation and dexamethasone pretreatment"
- J. CLIN. INVEST., vol. 77, 1986, pages 1903-1907, The American Society forClinical Investigation, Inc.; U. VETTER et al.: "Human fetal and adultchondrocytes. Effect of insulinlike growth factors I and II, insulin, andgrowth hormone on clonal growth"

## Description

### Field of the Invention

The invention relates to the field of steroid therapy and the reversing or preventing of adverse effects of steroid therapy with Insulin-like Growth Factor I (IGF-I). The invention further relates to compositions of combinations of steroids and IGF-I with or without other active agents.

### Background of the Invention

Steroids are used medicinally in a large number of conditions, exploiting a host of different properties. Unfortunately steroid therapy, especially long term steroid therapy, has a deleterious effect on cartilage and bone and wound repair. This is especially so for the steroids clinically utilized as anti-inflammatory agents (i.e the steroids with corticosteroid activity). These undesirable side effects are also present to varying degrees with other steroids in relation to the degree of corticosteroid activity possessed by such steroids. In addition, endogenous overproduction of steroids having corticosteroid activity can also have these deleterious effects.

### Objects of the Invention

Accordingly, it is an object of the invention to provide a method of inhibiting and/or reversing such adverse effects.

It is another object of the invention to provide compositions containing steroids having corticosteroid activity which composition as a whole is free of or has a lesser degree of such corticosteroid side effects.

It is a further object of the invention to safen the use of steroids having corticosteroid activity.

Yet another object of the invention is to provide for expanded application of therapy with steroids having corticosteroid activity to patients for which such therapy may have been contraindicated.

### Summary of the Invention

Surprisingly, these and other objects of the invention are realized by treating patients on steroid therapy, which steroids have corticosteroid activity, or patients who have suffered the adverse effects of corticosteroid activity set forth above with Insulin-like Growth Factor I (IGF-I).

### Detailed Description of the Invention

The instant invention relates to reversing undesirable effects of steroids having corticosteroid activity (whether exogenous steroids administered to the patient or the patient's own overproduction of endogenous steroid as in Cushing's disease) on cartilage, bone and wound repair.

IGF-I is a naturally occuring protein and can be isolated from a number of sources. It has also been made synthetically and by recombinant gene technology as seen in EP-A-123,228. For the present invention IGF-I and IGF-I active fragments of IGF-I of any source are suitable providing the immune response thereto is acceptably small, but most preferably the IGF-I used is chemically identical to that naturally found in the species being treated therewith or the IGF-I fragment having IGF-I activity is chemically identical to a fragment of the natural IGF-I of the species being treated. For purposes of this disclosure, IGF-I is intended to include fragments thereof unless the disclosure specifically provides otherwise. Where amounts of IGF-I are indicated, amounts of IGF-I fragments of approximately equipotent activity are intended to be included unless specifically indicated otherwise.

While any animal having had or undergoing steroid (having corticosteroid activity) therapy can benefit from the instant invention, it is primarily directed to treatment of mammals and more specifically human beings.

Accordingly, the present invention concerns a method of reducing adverse effects of corticosteroid activity in a mammal with endogenous overproduction of a corticosteroid or in need of administration of a steroid having corticosteroid activity comprising administering to said mammal a corticosteroid adverse effect reducing or preventing efficacious amount of IGF-I or an active fragment or analog thereof. Said corticosteroid adverse effect may be the result of an administration, e.g. chronic administration, of exogenous or of an overproduction of endogenous steroid having corticosteroid activity, e.g. a glucocorticoid.

While any such steroid therapy which results in negatively affecting cartilage or bone or wound repair or any other ill effects of inhibition of proteoglycan synthesis will benefit from the present invention, it is most favorably directed to use in osteoarthritis conditions.

Usually, an effective amount of IGF-I, when given parenterally (intravenously, subcutaneously, intramuscularly, etc.), is between 1 »g/kg/day up to 270 »g/kg/day, preferably 2 1/2 »g/kg/day up to 180 »g/kg/day, more preferably 5 »g/Kg/day up to 150 »g/kg/day, still more preferably 10 »g/kg/day up to 120 »g/Kg/day, even more preferably 20 »g/kg/day up to 100 »g/kg/day, still more preferably 30 »g/kg/day up to 90 »g/kg/day. When given continuously, such effective amount may be given in two or three doses spread over time such as by i.v. drip or subcutaneous injection(s) with the total daily dose being spread across the portion or the entire administration period. Typical continuous dosing is in the range of 2 1/2 »g/kg/hour up to 50 »g/kg/hour, preferably 5 »g/kg/hour up to 25 »g/kg/hour, although wider ranges of "continuous" administration amounts will be apparent to those of ordinary skill. When given by subcutaneous injection, it is most preferably administered from 3 times/week up to 3 times a day, preferably twice a week up to once or twice daily.

The IGF-I may also be administered orally in single or divided dally doses, most preferably in divided doses three times a day. When given orally, the usual effective daily dosage range is from 5 »g/kg/day up to 270 »g/kg/day, more preferably 15 »g/kg/day, up to 270 »g/kg/day, still more preferably 45 »g/kg/day up to 270 »g/kg/day. The most preferred oral dosage range is 15 »g/kg up to 90 »g/kg administered three times a day.

IGF-I may be administered together with a therapeutic steroid having corticosteroid activity or separately in steroid therapy or it may be administered alone as a means of reversing effects induced by steroids having corticosteroid activity administered to or endogenously overproduced in the patient. When administered separately from a steroid having corticosteroid activity being utilized therapeutically, IGF-I may be administered in any suitable formulation, by the same or different route as the steroid. Separate administration of IGF-I and a steroid having corticosteroid activity is preferred allowing for greater tailoring of dosages to individual needs. However, where suitable, a single formulation containing both a steroid having corticosteroid activity and IGF-I may be utilized for convenience purposes.

The specific dosage for a particular patient, of course, has to be adjusted to the degree of response, the route of administration, the individual weight and general condition of the patient to be treated, and is finally dependent upon the judgement of the treating physician.

The invention also concerns the use of IGF-I for the production of a pharmaceutical preparation for reducing or preventing adverse effects of corticosteroid activity on cartilage, bone and wound repair in a mammal in need of administration of a steroid having corticosteroid activity or with endogenous overproduction of a corticosteroid. Such a pharmaceutical praparation may comprise IGF-I and optionally other pharmaceutically active ingredients such as steroids having corticosteroid activity, for example those mentioned hereinafter. The steroids having corticosteroid activity are present in a pharmaceutical combination preparation of the invention in the usual amounts suitable for steroid therapy.

In general the pharmaceutical preparations for use in the present invention comprise an effective amount of IGF-I or an active fragment thereof together with a pharmaceutically, and if parenteral, a parenterally acceptable carrier or adjuvant. Compositions having an approximately 6 day supply typically contain from 0.1 mg to 15 mg, preferably 1 mg to 13 mg, more preferably 3 mg to 10 mg, most preferably 5 mg to 10 mg of IGF-I. The liquid carriers are typically sterile water, approximate physiologic saline, O.1 M acetic acid, 5% aqueous dextrose, etc.; preferably sterile water, physiologic saline, or 5% aqueous dextrose.

The carriers and adjuvants may be solid or liquid and may be organic or inorganic. The active compound and the compositions of the invention are preferably used in the form of preparations or infusions for parenteral (subcutaneous, intramuscular, intravenous, or intraarticular) administration. They may also be in oral form, i.e. tablets, capsules, oral solution, oral suspension, etc. when parenteral, such solutions are preferably isotonic aqueous solutions or suspensions which can be prepared before use, for example reconstituted from a lyophilised preparation. The pharmaceutical preparations may be sterilized and/or contain adjuvants, for example preservatives, stabilizers, wetting agents, emulsifiers, solubilizers, tonicity regulating salts, and/or buffers. Other adjuvants will of course be apparent to the ordinarily skilled formulation chemist.

The present pharmaceutical preparations are prepared from their constituent parts by techniques known in the art, for example lyophilization, dissolution, reconstitution, and suspension techniques, among others known to those of ordinary skill. They typically contain from 0.1% to 100% of active ingredient, especially in the case of solutions - 1% to 20% active ingredient and especially in the case of a lyophilizate - up to 100% of active ingredient.

While any steroid having any degree of corticosteroid activity can be safened with the instant invention, corticosteroids per se are the preferred focus. This class includes without limitation: beclomethasone, betamethasone, cortisone, desoxycorticosterone, dexamethasone, fludrocortisone, fluprednisolone, hydrocortisone, meprednisone, methylprednisolone, paramethasone, prednisolone, prednisone, and triamcinolone. Especially preferred for the purposes of the present invention are the steroids having corticosteroid activity administered intraarticularly in the treatment of osteoarthritis, such as, without limitation, dexamethasone.

The pharmaceutical preparations contemplated by the invention include, if desired, in addition to IGF-I and a suitable carrier, any pharmaceutically active agent used in conjunction with steroid therapy and/or other pharmacologically inactive but pharmaceutically desirable substances. For example, in many contexts steroids may be administered on a chronic basis in conjunction with another pharmaceutically active agent, such as an antiasthmatic agent. The two are given by different routes or require different dosage regimens such that combination in a single combination product is not possible. It is within the scope of the invention to provide compositions of IGF-I (its active fragment of an active analog of such IGF-I or such fragment) in combination with such other non-steroidal pharmaceutically active agents which are used in combination therapy with steroids. Hence, IGF-I in a combination product with a non-steroidal antiasthmatic is a combination within the invention which would be used in conjunction with steroid therapy.

Having fully described the invention, the following examples are presented to more clearly describe it.

### Examples 1-3: Dry ampules of IGF-I

Sterile, filtered 1% (w/v) aqueous solution of IGF-I is added, in the amount indicated to the respective dry ampules the solution is then lyophilized to result in the dry ampules to be reconstituted shortly before use with the indicated amount of sterile water, physiologic saline, 0.1 M acetic acid, or 5% aqueous dextrose. Each vial is sufficient for a 6 day course of treatment for the intended patient.

| | Ex 1 | Ex 2 | Ex 3 |
|---|---|---|---|
| ampule size | 5 ml | 8 ml | 50 ml |
| IGF-I fill volume | 1 ml | 5 ml | 30 ml |
| Reconstitution Volume | 1 ml | 5 ml | 30 ml |

### Example 4: Effect of Drugs on proteoglycan synthesis by bovine articular chondrocytes in the presence and absence of IGF-I

| Drug | No IGF | IGF-I (100 ng/ml) |
|---|---|---|
| Dexamethasone nM | S-GAG »g/ml | |
| 0 | 11.2 ± 0.2 | 14.5 ± 0.5 |
| 0.1 | 9.4 ± 0.2 | 11.8 ± 0.7 |
| 1 | 8.5 ± 0.2 | 11.3 ± 0.3 |
| 10 | 7.3 ± 0.4 | 9.9 ± 0.1 |
| 100 | 7.3 ± 0.1 | 9.3 ± 0.2 |

Bovine articular chondrocytes were prepared according to the procedure of Kuettner et al. After a 2 day growth the medium was changed and fresh medium containing drug and/or IGF-I was added. After a 3 day exposure the medium was harvested and the sulfated glycosaminoglycan (S-GAG), a measure of proteoglycan synthesis, was determined by a dye binding assay according to Farnsdale et al. Values are the mean ± S.E.M. from 4 cultures.

### Examples 5 to 10: Combinations of IGF-I and steroid

Sterile, filtered 1% (w/v) aqueous solution of IGF-I is added, in the amount indicated, along with the respective steroid, in the amount indicated in dry ampules. The solution is then lyophilized to result in the dry ampules to be reconstituted shortly before use with the indicated amount of sterile water, physiologic saline, or 5% aqueous dextrose.

| | Ex 5 | Ex 6 | Ex 7 |
|---|---|---|---|
| ampule size | 5 ml | 8 ml | 50 ml |
| IGF-I fill volume | 1 ml | 5 ml | 30 ml |
| triamcinolone | 10 mg | 50 mg | 300 mg |
| Reconstitution Volume | 1 ml | 5 ml | 30 ml |

| | Ex 8 | Ex 9 | Ex 10 |
|---|---|---|---|
| ampule size | 5 ml | 8 ml | 50 ml |
| IGF-I fill volume | 1 ml | 5 ml | 30 ml |
| dexamethasone | 10 mg | 50 mg | 300 mg |
| Reconstitution Volume | 1 ml | 5 ml | 30 ml |

### References:

Kuettner, K. E. et al., J. Cell Biol. 93:743, 1982.
Farnsdale, C. A. et al., Connect. Tissue Res. 9:247, 1982.

## Claims (Claims for the following Contracting State(s): AT, BE, CH, DE, DK, FR, GB, IT, LI, LU, NL, SE)

1. Use of IGF-I for the production of a pharmaceutical preparation for reducing or preventing an adverse effect of corticosteroid activity on cartilage, bone and wound repair in a mammal.

2. Use according to claim 1 wherein said adverse effect is the result of administration of exogenous steroid having corticosteroid activity.

3. Use according to claim 1 wherein said adverse effect is the result of endogenous overproduction of steroid having corticosteroid activity.

4. Use according to claim 2 wherein said adverse effect is the result of chronic administration of exogenous steroid having corticosteroid activity.

5. Use according to claim 2 wherein the exogenous steroid is selected from beclomethasone, betamethasone, cortisone, desoxycorticosterone, dexamethasone, fludrocortisone, fluprednisolone, hydrocortisone, meprednisone, methylprednisolone, paramethasone, prednisolone, prednisone, and triamcinolone.

6. Use according to claim 1 wherein said pharmaceutical preparation comprises a steroid having corticosteroid activity and IGF-I or an active fragment or analog thereof in a single formulation.

7. Use according to claim 1 wherein said pharmaceutical preparation comprises a steroid having corticosteroid activity and IGF-I in different formulations for the administration by the same or by different routes.

8. Use according to claim 2 for the production of a pharmaceutical preparation for reducing or preventing an adverse effect of exogenous corticosteroid administered in the treatment of osteoarthritis.

9. Use according to claim 1 wherein the IGF-I corresponds to the natural form of the IGF-I of the species being treated.

10. Use according to claim 9 wherein the IGF-I corresponds to human IGF-I.

11. Use according to claim 1 wherein the IGF-I does not correspond to the natural form of the IGF-I of the species being treated.

12. Use according to claim 1 wherein the IGF-I is prepared by recombinant techniques.

13. A therapeutic composition comprising a pharmaceutically effective amount of a steroid having corticosteroid activity and a corticosteroid adverse effect reducing or preventing effective amount of IGF-I.

## Claims (Claims for the following Contracting State(s): ES, GR)

1. Use of IGF-I for the production of a pharmaceutical preparation for reducing or preventing an adverse effect of corticosteroid activity on cartilage, bone and wound repair in a mammal.

2. Use according to claim 1 wherein said adverse effect is the result of administration of exogenous steroid having corticosteroid activity.

3. Use according to claim 1 wherein said adverse effect is the result of endogenous overproduction of steroid having corticosteroid activity.

4. Use according to claim 2 wherein said adverse effect is the result of chronic administration of exogenous steroid having corticosteroid activity.

5. Use according to claim 2 wherein the exogenous steroid is selected from beclomethasone, betamethasone, cortisone, desoxycorticosterone, dexamethasone, fludrocortisone, fluprednisolone, hydrocortisone, meprednisone, methylprednisolone, paramethasone, prednisolone, prednisone, and triamcinolone.

6. Use according to claim 1 wherein said pharmaceutical preparation comprises a steroid having corticosteroid activity and IGF-I or an active fragment or analog thereof in a single formulation.

7. Use according to claim 1 wherein said pharmaceutical preparation comprises a steroid having corticosteroid activity and IGF-I in different formulations for the administration by the same or by different routes.

8. Use according to claim 2 for the production of a pharmaceutical preparation for reducing or preventing an adverse effect of exogenous corticosteroid administered in the treatment of osteoarthritis.

9. Use according to claim 1 wherein the IGF-I corresponds to the natural form of the IGF-I of the species being treated.

10. Use according to claim 9 wherein the IGF-I corresponds to human IGF-I.

11. Use according to claim 1 wherein the IGF-I does not correspond to the natural form of the IGF-I of the species being treated.

12. Use according to claim 1 wherein the IGF-I is prepared by recombinant techniques.

## Patentansprüche (Patentansprüche für folgende(n) Vertragsstaat(en): AT, BE, CH, DE, DK, FR, GB, IT, LI, LU, NL, SE)

1. Verwendung von IGF-I zur Herstellung eines pharmazeutischen Präparats zur Verringerung oder Verhütung einer ungünstigen Wirkung einer Corticosteroid-Aktivität auf Knorpel, Knochen und Wundheilung bei einem Säuger.

2. Verwendung nach Anspruch 1, worin die ungünstige Wirkung das Ergebnis der Verabreichung eines exogenen Steroids mit Corticosteroid-Aktivität ist.

3. Verwendung nach Anspruch 1, worin die ungünstige Wirkung das Ergebnis einer endogenen Überproduktion eines Steroids mit Corticosteroid-Aktivität ist.

4. Verwendung nach Anspruch 2, worin die ungünstige Wirkung das Ergebnis einer chronischen Verabreichung eines exogenen Steroids mit Corticosteroid-Aktivität ist.

5. Verwendung nach Anspruch 2, worin das exogene Steroid aus Beclomethason, Betamethason, Cortison, Desoxycorticosteron, Dexamethason, Fludrocortison, Fluprednisolon, Hydrocortison, Meprednison, Methylprednisolon, Paramethason, Prednisolon, Prednison und Triamcinolon ausgewählt wird.

6. Verwendung nach Anspruch 1, worin das pharmazeutische Präparat ein Steroid mit Corticosteroid-Aktivität und IGF-I oder ein aktives Fragment oder Analogon davon in einer Einzelformulierung umfaßt.

7. Verwendung nach Anspruch 1, worin das pharmazeutische Präparat ein Steroid mit Corticosteroid-Aktivität und IGF-I in verschiedenen Formulierungen zur Verabreichung auf dem gleichen Weg oder auf verschiedenen Wegen umfaßt.

8. Verwendung nach Anspruch 2 zur Produktion eines pharmazeutischen Präparats zur Verringerung oder Verhütung einer ungünstigen Wirkung eines exogenen Corticosteroids, das zur Behandlung von Osteoarthritis verabreicht wurde.

9. Verwendung nach Anspruch 1, worin der IGF-I der natürlichen Form des IGF-I der behandelten Art entspricht.

10. Verwendung nach Anspruch 9, worin der IGF-I menschlichem IGF-I entspricht.

11. Verwendung nach Anspruch 1, worin der IGF-I nicht der natürlichen Form des IGF-I der behandelten Art entspricht.

12. Verwendung nach Anspruch 1, worin der IGF-I durch rekombinante Techniken hergestellt worden ist.

13. Therapeutische Zusammensetzung, umfassend eine pharmazeutisch wirksame Menge eines Steroids mit Corticosteroid-Aktivität und eine die ungünstige Wirkung des Corticosteroids verringernde oder verhütende wirksame Menge an IGF-I.

## Patentansprüche (Patentansprüche für folgende(n) Vertragsstaat(en): ES, GR)

1. Verwendung von IGF-I zur Herstellung eines pharmazeutischen Präparats zur Verringerung oder Verhütung einer ungünstigen Wirkung einer Corticosteroid-Aktivität auf Knorpel, Knochen und Wundheilung bei einem Säuger.

2. Verwendung nach Anspruch 1, worin die ungünstige Wirkung das Ergebnis der Verabreichung eines exogenen Steroids mit Corticosteroid-Aktivität ist.

3. Verwendung nach Anspruch 1, worin die ungünstige Wirkung das Ergebnis einer endogenen Überproduktion eines Steroids mit Corticosteroid-Aktivität ist.

4. Verwendung nach Anspruch 2, worin die ungünstige Wirkung das Ergebnis einer chronischen Verabreichung eines exogenen Steroids mit Corticosteroid-Aktivität ist.

5. Verwendung nach Anspruch 2, worin das exogene Steroid aus Beclomethason, Betamethason, Cortison, Desoxycorticosteron, Dexamethason, Fludrocortison, Fluprednisolon, Hydrocortison, Meprednison, Methylprednisolon, Paramethason, Prednisolon, Prednison und Triamcinolon ausgewählt wird.

6. Verwendung nach Anspruch 1, worin das pharmazeutische Präparat ein Steroid mit Corticosteroid-Aktivität und IGF-I oder ein aktives Fragment oder Analogon davon in einer Einzelformulierung umfaßt.

7. Verwendung nach Anspruch 1, worin das pharmazeutische Präparat ein Steroid mit Corticosteroid-Aktivität und IGF-I in verschiedenen Formulierungen zur Verabreichung auf dem gleichen Weg oder auf verschiedenen Wegen umfaßt.

8. Verwendung nach Anspruch 2 zur Produktion eines pharmazeutischen Präparats zur Verringerung oder Verhütung einer ungünstigen Wirkung eines exogenen Corticosteroids, das zur Behandlung von Osteoarthritis verabreicht wurde.

9. Verwendung nach Anspruch 1, worin der IGF-I der natürlichen Form des IGF-I der behandelten Art entspricht.

10. Verwendung nach Anspruch 9, worin der IGF-I menschlichem IGF-I entspricht.

11. Verwendung nach Anspruch 1, worin der IGF-I nicht der natürlichen Form des IGF-I der behandelten Art entspricht.

12. Verwendung nach Anspruch 1, worin der IGF-I durch rekombinante Techniken hergestellt worden ist.

## Revendications (Revendications pour l'(les) Etat(s) contractant(s) suivant(s): AT, BE, CH, DE, DK, FR, GB, IT, LI, LU, NL, SE)

1. Utilisation d'IGF-I pour la production d'une préparation pharmaceutique pour la réduction ou l'inhibition d'un effet indésirable induit par une activité de corticostéroïde sur le cartilage, les os et la cicatrisation des plaies chez un mammifère.

2. Utilisation selon la revendication 1, dans laquelle le dit effet indésirable est le résultat d'une administration de stéroïde exogène ayant une activité de corticostéroïde.

3. Utilisation selon la revendication 1, dans laquelle le dit effet indésirable est le résultat de la surproduction endogène de stéroïde ayant une activité de corticostéroïde.

4. Utilisation selon la revendication 2, dans laquelle le dit effet indésirable est le résultat d'une administration chronique de stéroïde exogène ayant une activité de corticostéroïde.

5. Utilisation selon la revendication 2, dans laquelle le stéroïde exogène est choisi parmi la béclométhasone, la bêtaméthasone, la cortisone, la désoxycorticostérone, la dexaméthasone, la fludrocortisone, la fluprednisolone, l'hydrocortisone, la méprednisone, la méthylprednisolone, la paraméthasone, la prednisolone, la prednisone et la triamcinolone.

6. Utilisation selon la revendication 1, dans laquelle la dite préparation pharmaceutique comprend un stéroïde ayant une activité de corticostéroïde et de l'IGF-I ou un fragment actif ou analogue de celui-ci en une formulation unique.

7. Utilisation selon la revendication 1, dans laquelle la dite préparation pharmaceutique comprend un stéroïde ayant une activité de corticostéroïde et de l'IGF-I dans des formulations différentes pour l'administration par la même voie ou des voies différentes.

8. Utilisation selon la revendication 2 pour la production d'une préparation pharmaceutique pour la réduction ou l'inhibition d'un effet indésirable induit par des corticostéroïdes administrés dans le traitement de l'ostéo-arthrite.

9. Utilisation selon la revendication 1, dans laquelle l'IGF-I correspond à la forme naturelle de l'IGF-I de l'espèce en traitement.

10. Utilisation selon la revendication 9, dans laquelle l'IGF-I correspond à l'IGF-I humain.

11. Utilisation selon la revendication 1, dans laquelle l'IGF-I ne correspond pas à la forme naturelle de l'IGF-I de l'espèce en traitement.

12. Utilisation selon la revendication 1, dans laquelle l'IGF-I est préparé par des techniques de recombinaison génétique.

13. Composition thérapeutique comprenant une quantité pharmaceutiquement efficace d'un stéroïde ayant une activité de corticostéroïde et une quantité d'IGF-I efficace pour réduire ou inhiber l'effet indésirable des corticostéroïdes.

## Revendications (Revendications pour l'(les) Etat(s) contractant(s) suivant(s): ES, GR)

1. Utilisation d'IGF-I pour la production d'une préparation pharmaceutique pour la réduction ou l'inhibition d'un effet indésirable induit par une activité de corticostéroïde sur le cartilage, les os et la cicatrisation des plaies chez un mammifère.

2. Utilisation selon la revendication 1, dans laquelle le dit effet indésirable est le résultat d'une administration de stéroïde exogène ayant une activité de corticostéroïde.

3. Utilisation selon la revendication 1, dans laquelle le dit effet indésirable est le résultat de la surproduction endogène de stéroïde ayant une activité de corticostéroïde.

4. Utilisation selon la revendication 2, dans laquelle le dit effet indésirable est le résultat d'une administration chronique de stéroïde exogène ayant une activité de corticostéroïde.

5. Utilisation selon la revendication 2, dans laquelle le stéroïde exogène est choisi parmi la béclométhasone, la bêtaméthasone, la cortisone, la désoxycorticostérone, la dexaméthasone, la fludrocortisone, la fluprednisolone, l'hydrocortisone, la méprednisone, la méthylprednisolone, la paraméthasone, la prednisolone, la prednisone et la triamcinolone.

6. Utilisation selon la revendication 1, dans laquelle la dite préparation pharmaceutique comprend un stéroïde ayant une activité de corticostéroïde et de l'IGF-I ou un fragment actif ou analogue de celui-ci en une formulation unique.

7. Utilisation selon la revendication 1, dans laquelle la dite préparation pharmaceutique comprend un stéroïde ayant une activité de corticostéroïde et de l'IGF-I dans des formulations différentes pour l'administration par la même voie ou des voies différentes.

8. Utilisation selon la revendication 2 pour la production d'une préparation pharmaceutique pour la réduction ou l'inhibition d'un effet indésirable induit par des corticostéroïdes administrés dans le traitement de l'ostéo-arthrite.

9. Utilisation selon la revendication 1, dans laquelle l'IGF-I correspond à la forme naturelle de l'IGF-I de l'espèce en traitement.

10. Utilisation selon la revendication 9, dans laquelle l'IGF-I correspond à l'IGF-I humain.

11. Utilisation selon la revendication 1, dans laquelle l'IGF-I ne correspond pas à la forme naturelle de l'IGF-I de l'espèce en traitement.

12. Utilisation selon la revendication 1, dans laquelle l'IGF-I est préparé par des techniques de recombinaison génétique.
